# EUROPEAN PATENT SPECIFICATION

(11) **EP 2 997 881 B1**
(45) Date of publication and mention of the grant of the patent: **12.07.2023**
(21) Application number: 15185730.7
(22) Date of filing: 17.09.2015
(51) Int. Cl.: A61B 3/12, A61B 3/15, A61B 3/16, A61B 3/00, A61B 3/117, A61B 3/135

(54) **OPHTHALMIC APPARATUS**
OPHTHALMISCHE VORRICHTUNG
APPAREIL OPHTALMIQUE

(30) Priority: 20.09.2014 JP 2014192058
(43) Date of publication of application: 23.03.2016
(73) Proprietor: NIDEK CO., LTD, Gamagori-shi Aichi 443-0038 (JP)
(72) Inventor: SAKASHITA, Yusuke, Gamagori-shi, Aichi 443-0038 (JP); SHIMIZU, Kazunari, Gamagori-shi, Aichi 443-0038 (JP)
(74) Representative: Prüfer & Partner mbB Patentanwälte · Rechtsanwälte

(56) References cited:
- EP-A1- 2 382 912
- EP-A1- 2 586 361
- EP-A1- 2 668 893
- JP-A- 2004 329 872
- JP-A- 2008 011 878
- US-A1- 2010 165 293

## Description

### BACKGROUND

This disclosure relates to an ophthalmic apparatus provided with a cross-section imaging optical system for photographing a cross-sectional image (a cross-sectional image) of an anterior segment with slit light.

EP 2 586 361 A1 discloses a intraocular lens power determination apparatus for determining a highly precise IOL power including an anterior segment imaging device for obtaining a cross-sectional image of an anterior segment by detecting reflection from the anterior segment of an examiner's eye; and a power calculation unit obtaining an offset distance from a front surface of a lens to a point of contact of a Zinn's zonule with the lens based on the anterior segment cross-sectional image obtained by the anterior segment imaging device.

JP 2008 011878 A discloses an ophthalmologic apparatus comprising an intraocular pressure measuring means for measuring the intraocular pressure of an eye to be examined and a cornea thickness measuring means for projecting a luminous flux to the cornea of the eye to be examined, detecting the reflected light and measuring the thickness of the cornea.

One example of an ophthalmic apparatus provided with an imaging optical system for photographing or capturing a cross-sectional image of an anterior segment of an eye with slit light is known as a non-contact tonometer having a corneal thickness measuring function (see Patent Document 1). In this case, the cross-section imaging optical system is aligned with respect to a corneal center of an examinee's eye and then a corneal cross-sectional image is photographed. Thereafter, the corneal thickness is measured based on the corneal cross-sectional image (First conventional technique).

Further, there is also known an apparatus consisting of a single Scheimpflug's camera (see Patent Document 2). Such an apparatus is able to simultaneously photograph the entire anterior segment including the cornea, the lens, an iridocorneal area, and others (Second conventional technique).

### Related Art Documents

### Patent Documents

Patent Document 1: JP-A-2014-068829
Patent Document 2: JP-A-06-014885(1994)

### SUMMARY

However, in the case of the first conventional technique, the photographing range is limited due to the influence of a nozzle and others and thus only a corneal cross-sectional image is photographed. Specifically, an image of an iridocorneal area of an anterior segment could not be obtained. In the case of the second conventional technique, an image of an iridocorneal area of an anterior segment could be obtained; however, this may lead to upsizing and complication of an optical system, and other defects.

The present disclosure has been made to address the above problems and has a purpose to provide an ophthalmic apparatus capable of appropriately obtaining a cross-sectional image of an iridocorneal area of an anterior segment.

One example of the present disclosure to achieve the above mentioned purpose is an ophthalmic apparatus according to claim 1. Further developments of the present disclosure are given in the dependent claims.

According to the present disclosure, it is possible to appropriately obtain a cross-sectional image of an iridocorneal area of an anterior segment.

### BRIEF DESCRIPTION OF THE DRAWINGS

FIG. 1 is an external view of an ophthalmic apparatus in a first embodiment;
FIG. 2 is a schematic configuration view of an optical system placed (housed) in a measurement unit;
FIG. 3 is a diagram showing a positional relationship between the measuring unit and a cross-section imaging unit and showing a schematic configuration of an optical system placed in the cross-section imaging unit;
FIG. 4 is a diagram showing a schematic configuration of an intraocular pressure measuring unit and a control system of the ophthalmic apparatus;
FIG. 5 is a diagram showing one example of an observation screen displayed on a monitor in a corneal thickness measuring mode and an intraocular pressure measuring mode;
FIG. 6 is a diagram showing one example of an observation screen displayed on a monitor in an iridocorneal-area photographing mode;
FIG. 7 is a diagram showing an observation screen displayed on the monitor in the iridocorneal-area photographing mode, on which a live image of an iridocorneal-area cross-sectional image is displayed together with a live image of an anterior-segment front image;
FIG. 8 is a diagram showing one example of a display pattern of a photographed image (a still image) of the iridocorneal-area cross-sectional image;
FIG. 9 is an observation image displayed on the monitor in the iridocorneal-area photographing mode, on which a thumbnail of the iridocorneal-area cross-sectional image is displayed; and
FIG. 10 is a diagram showing one example of a screen layout whereby a photographing result and a measurement result in each mode are merged and displayed.

### DETAILED DESCRIPTION OF THE EXEMPLARY EMBODIMENTS

A typical embodiment of an ophthalmic apparatus according to the present disclosure will be explained below referring to the accompanying drawings. An ophthalmic apparatus 100 in a first embodiment is arranged to obtain a cross-sectional image of an anterior segment of an examinee's eye. In the first embodiment, particularly, the ophthalmic apparatus 100 is able to obtain a cross-sectional image in a range including an iridocorneal area (which is a joint portion between the cornea and the iris) of the examinee's eye. It is to be noted that the following explanation is given assuming that the ophthalmic apparatus 100 is a combined apparatus of a tonometer for measuring the intraocular pressure of an examinee's eye E in non-contact relation and a pachymeter for measuring the corneal thickness of the eye E in non-contact relation.

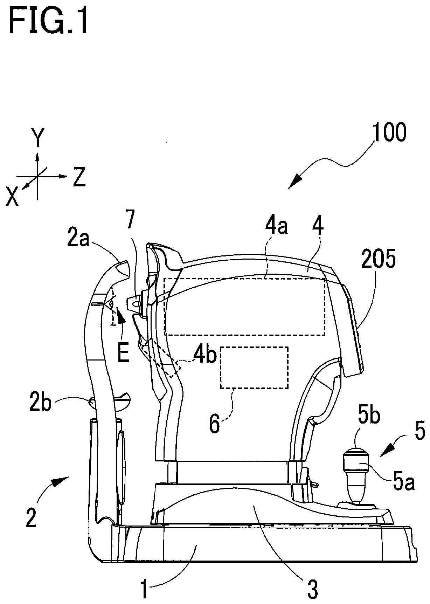

Referring to FIG. 1, firstly, an external configuration of the ophthalmic apparatus 100 will be explained. The ophthalmic apparatus 100 includes, as shown in FIG. 1, a table 1, a face (head) support unit 2, a movable base 3, an electrical moving mechanism 6, and a main unit (an optometry unit) 4, and a joystick 5.

The face support unit 2 is attached to the table 1. The face support unit 2 includes a forehead rest 2a for supporting the forehead of an examinee putted in contact therewith and a chin rest (a chin table) 2b for supporting the chin of the examinee in contact therewith.

The movable base 3 is placed to be movable on the table 1 in each of an X direction (i.e., a right and left direction) and a Z direction (i.e., a working distance direction and a back and forth direction). In the first embodiment, at least either the table 1 or the movable base 3 has a mechanical moving mechanism (e.g., a sliding mechanism) to move the movable base 3.

The main unit 4 includes a measuring unit 4a and a cross-section imaging unit (an anterior-segment cross-section imaging unit) 4b. These units 4a and 4b will be explained in detail later. The main unit 4 is movable three-dimensionally with respect to the examinee's eye E. Specifically, the main unit 4 is movable with respect to the movable base 3. Concretely, by driving of the electrical moving mechanism 6, the main unit 4 is moved with respect to the movable base 3.

The joystick 5 is an operating member to be operated by an examiner for manual alignment, for example, to manually adjust a positional relationship between the examinee's eye E and the main unit 4. In the first embodiment, a tilting operation of the joystick 5 causes the movable base 3 to move over the table 1 in the X direction and the Z direction. By a rotating operation of a rotary knob 5a of the joystick 5, the electrical moving mechanism 6 is driven to move the main unit 4 in the Y direction (i.e., an up and down direction). The electrical moving mechanism 6 is a unit for moving the main unit 4 in each of the X, Y, and Z directions. The electrical moving mechanism 6 is utilized not only for vertical movement of the main unit 4 by manual operation but also for automatic alignment and tracking of the main unit 4 with respect to the examinee's eye E, and others. When alignment is completed, the measuring unit 4a is located in front of the examinee's eye E. In this case, the cross-section imaging unit 4b is placed in an oblique lower position relative to the examinee's eye E. In the first embodiment, the head of the joystick 5 is provided with a measuring start switch 5b.

In the first embodiment, a housing surface of the main unit 4 on a side facing the examiner is attached with a monitor 205. The examiner performs operations of the apparatus while observing the monitor 205. This monitor 205 in the present embodiment is a touch panel.

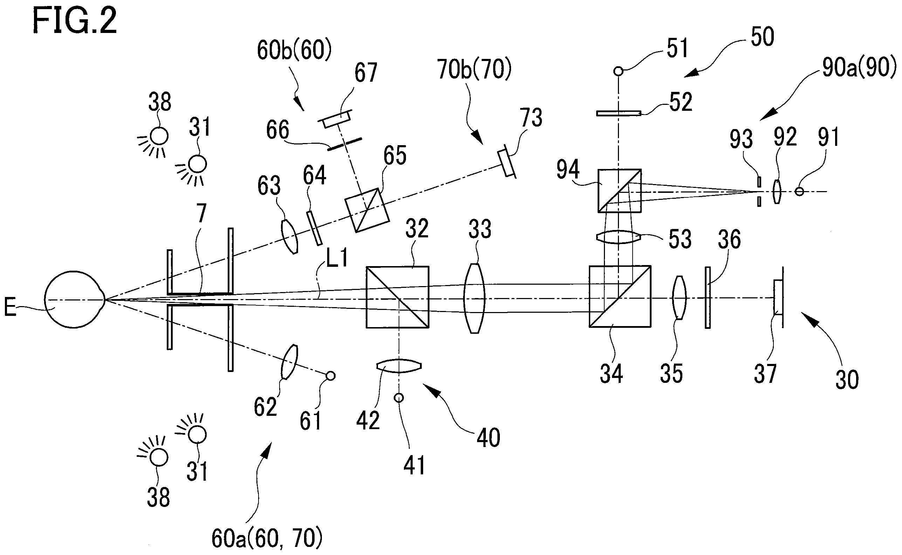

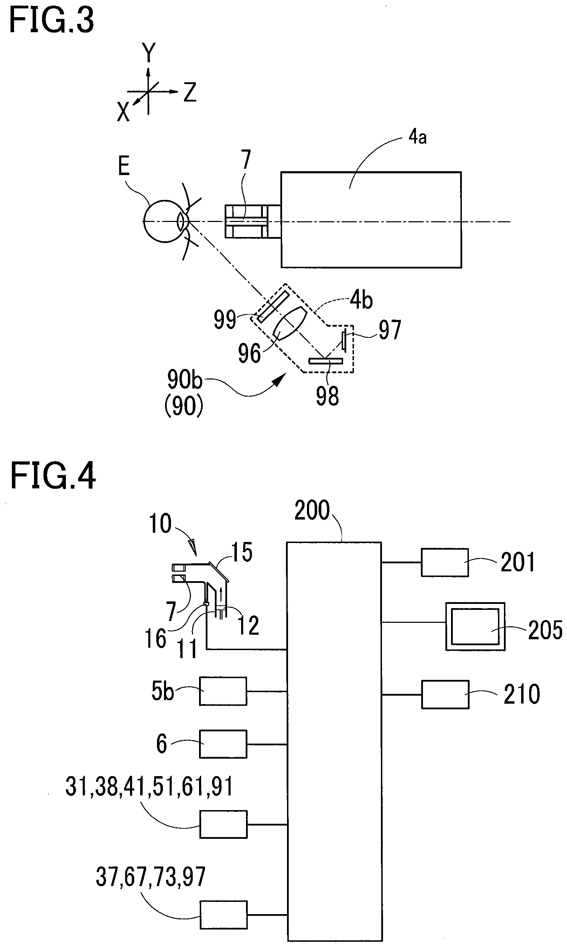

Optical systems of the ophthalmic apparatus 100 will be explained below referring to FIGs. 2 and 3. The optical systems of the ophthalmic apparatus 100 are housed in the units 4a and 4b of the main unit 4. The ophthalmic apparatus 100 has an anterior-segment cross-section imaging optical system 90 (hereinafter, referred to as a cross-section imaging optical system) as an optical system for obtaining a cross-sectional image of the anterior segment. The cross-section imaging optical system 90 in the first embodiment includes a light projecting optical system 90a (see FIG. 2) and a light receiving optical system 90b (see FIG. 3). As shown in FIG. 2, furthermore, the ophthalmic apparatus 100 is provided with a front observation optical system 30 (an anterior-segment front imaging optical system) 30, an alignment optical system 40, a fixation optical system 50, a corneal deformation detecting optical system 60 (60a and 60b in FIG. 1), and a working distance detecting optical system 70 (60a and 70b in FIG. 1). The ophthalmic apparatus 100 in the first embodiment further includes an intraocular pressure measuring unit 10 (see FIG. 4). The intraocular pressure measuring unit 10 is housed in the measuring unit 4a.

The front observation optical system 30 is used to observe and photograph an anterior-segment front image of the examinee's eye E. This front observation optical system 30 is provided with for example a beam splitter 32, an objective lens 33, a beam splitter 34, an imaging lens 35, a filter 36, and an imaging element (a two-dimensional imaging element) 37, which are arranged on an optical axis L1. The front observation optical system 30 is configured to illuminate the anterior segment with illumination light which is one of infrared light emitted from a light source 31 and visible light emitted from a light source 38. In the first embodiment, anterior-segment reflection light of the illumination light reflected by the anterior segment passes along an optical path from the beam splitter 32 to the filter 36 to form an anterior-segment front image on an imaging element 37. In the first embodiment, the filter 36 allows passage of the light from the light sources 31 and 38 and the light from a light source 41 for alignment. The filter 36 also has a property of not allowing passage of the light from a light source 61 for detection of corneal deformation. The anterior-segment front image formed on the imaging element 37 is displayed on the monitor 205.

The alignment optical system 40 includes for example a light source 41 and a projection lens 42. In the first embodiment, the alignment optical system 40 shares the elements arranged on the optical axis L1, the beam splitter 32 to the imaging element 37, with the front observation optical system 30. In the first embodiment, the light source 41 emits infrared light to form an alignment index. The infrared light from the light source 41 passes through the projection lens 42 and is reflected by the beam splitter 32 to irradiate the examinee's eye E from front. As a result, a corneal luminescent spot (a cornea reflection image) is formed at a corneal vertex. The luminescent spot is formed on the imaging element 37 through the beam splitter 32 to the filter 36. Accordingly, the luminescent spot is captured together with the anterior-segment front image. The luminescent spot is utilized as an alignment index for alignment in up-and-down and right-and-left directions, the details of which will be mentioned later.

The fixation optical system 50 includes a visible light source (a fixation lamp) 51, a fixation target 52, a dichroic mirror 94, and a projection lens 53. In the present embodiment, the fixation optical system 50 shares the elements arranged on the optical axis Ll, the beam splitter 34 to the beam splitter 32, with the front observation optical system 30 and others. When the visible light source 51 is turned on, an image of the fixation target 52 is presented to the examinee's eye E from front. This fixes the visual line of the eye E frontward to the fixation point. In this case, the optical axis L1 is used as a fixation optical axis. The visible light source 51 may be for example an LED, a laser, or other light sources. Further, the visible light source 51 may also be for example a two-dimensional indicator such as a liquid crystal display as well as a pattern light source such as a spot light source, a slit light source, and a ring light source.

The corneal deformation detecting optical system 60 is used to detect a deformed state of a cornea. This optical system 60 includes a light projecting optical system 60a and a light receiving optical system 60b. The light projecting optical system 60a irradiates illumination light toward a cornea from an oblique direction. The light projecting optical system 60a includes a light source 61 and a projection lens 62. On the other hand, the light receiving optical system 60b receives, at the photodetector 67, cornea reflection light of the illumination light emitted from the light projecting optical system 60a and reflected by the cornea. The light receiving optical system 60b includes a lens 63, a filter 64, a beam splitter 65, and a pinhole plate 66 in addition to the photodetector 67. In the present embodiment, the corneal deformation detecting optical system 60 is placed so that an amount of light received at the photodetector 67 is largest when the examinee's eye E is in a predetermined deformed state (e.g., a flattened state). Accordingly, the deformation of the examinee's eye E is detected based on a light receiving signal output from the photodetector 67 in relation to the light received amount.

Deforming the examinee's eye E is performed by blowing compressed fluid (e.g., compressed air) from a nozzle 7 of the intraocular pressure measuring unit 10 to the examinee's eye E. The ophthalmic apparatus 100 in the first embodiment performs measurement of the intraocular pressure based on a deformed amount of the eye E detected by use of the photodetector 67 at the time of blowing the compressed fluid.

Herein, the structure of the intraocular pressure measuring unit 10 will be explained referring to FIG. 4. This intraocular pressure measuring unit 10 is provided with a fluid blowing mechanism configured to move a piston 12 in a cylinder 11 by a solenoid (not shown) in a compression direction to thereby inject compressed air to the examinee's eye E through the nozzle 7. The intraocular pressure measuring unit 10 includes a pressure sensor 16 for detecting the pressure in the cylinder 11. Based on a detection signal from the pressure sensor 16, the intraocular pressure is measured. The cylinder 11 has a transparent glass plate 15 and a rear wall. The glass plate 15 transmits the light projected or received by the foregoing optical systems without blocking.

Returning to FIG. 2, the optical systems will be continuously explained. The working-distance detecting optical system 70 is utilized for alignment in a working distance direction (the Z direction). This working-distance detecting optical system 70 shares the light projecting optical system 60a with the corneal deformation detecting optical system 60. This optical system 70 includes a light receiving optical system 70b. This light receiving optical system 70b includes a position detecting element 73. As the position detecting element 73, for example, a one-dimensional position detecting element such as a PSD and a line sensor may be used. The light emitted from the light projecting optical system 60a forms an index image on a cornea. The cornea reflection light of the light emitted by the light projecting optical system 60a passes through the lens 64 and the beam splitter 65 and then enters the position detecting element 73. The light receiving position on the position detecting element 73 is displaced according to the positional relationship in the working distance direction (Z direction) between the measuring unit 10 and the cornea of the examinee's eye E. As a result, the alignment state in the Z direction is detected based on an output signal from the position detecting element 73 (that is, working distance information is obtained).

In the first embodiment, as shown in FIG. 2, the light projecting optical system 90a of the cross-section imaging optical system 90 is provided in the measuring unit 4a. The light projecting optical system 90a projects slit light of the light emitted from a light source 91 to the anterior segment of the examinee's eye E to consequently form a light section on the anterior segment. In the present embodiment, the light section passing through the optical axis L1 is formed on the anterior segment. In this case, the optical axis L1 is used as a light projecting optical axis of the slit light. In the first embodiment, the light section is formed horizontally in a right and left direction with respect to the anterior segment. In the first embodiment, the light projecting optical system 90a includes the light source 91, a condensing lens 92, a slit plate 93, and a dichroic mirror 94. Further, the light projecting optical system 90a shares the elements from the projection lens 53 to the beam splitter 32 with the fixation optical system 50. In the first embodiment, the light projecting optical system 90a projects slit light onto the examinee's eye E through the nozzle 7.

In the first embodiment, as shown in FIG. 3, the light receiving optical system 90b of the cross-section imaging optical system 90 is provided in the cross-section imaging unit 4b. The light receiving optical system 90b in the first embodiment includes an imaging lens 96, a total reflection mirror 98, and a detector (a two-dimensional imaging element) 97 and is configured to capture an anterior-segment cross-sectional image from an oblique direction to the examinee's eye.

The detector 97 has an imaging plane placed in a substantially conjugate position to the anterior segment. The imaging lens 96 and the total reflection mirror 98 direct anterior-segment scattered light (anterior-segment reflection light) obtained by scattering at the anterior segment to the imaging element 97. As a result, the imaging element 97 receives the anterior-segment scattered light obtained by scattering of the light section at the anterior segment. The light receiving optical system 90b has an imaging optical axis inclined with respect to the light projecting optical axis of the light projecting optical system 90a and is placed so that the light section of the projected image by the light projecting optical system 90a, the lens system including the cornea (the cornea and the imaging lens 96), and an imaging plane of the imaging element 97 are arranged in a Scheimpflug relationship. A filter 99 is placed on the front side of the lens 96 (on an examinee's eye E side) and transmits only the light (blue light) emitted from the light source 91 and used for imaging the anterior-segment cross-sectional image.

Next, a control system of the ophthalmic apparatus 100 will be explained referring to FIG. 4. A control unit 200 is a processor for controlling the entire apparatus and calculating measurement results. The control unit 200 is electrically connected to a memory 201 and the monitor 205 through a bus and others. The memory 201 has stored various control programs. The memory 201 also may store images photographed by the ophthalmic apparatus 100. The control unit 200 is also electrically connected to each of the light sources (namely, the light sources 31, 38, 41, 51, 61, and 91) of the ophthalmic apparatus 100 and each of the detectors (namely, the imaging element 37, the photodetector 67, the position detecting element 73, and the detector 97).

The control unit 200 is also connected to the electrical moving mechanism 6. This electrical moving mechanism 6 is operative to move the main unit 4 based on a control signal from the control unit 200 in each of the X, Y, and Z directions.

The control unit 200 is further connected to an operating unit 210 which is an input device to be operated by an examiner. The operating unit 210 is for example a switch, a keyboard, a pointing device (e.g., a mouse and a touch panel), or the like. The control unit 200 receives a signal output from the operating unit 210 operated by the examiner as a command from the examiner.

The monitor 205 is used as an information output device and controlled by the control unit 200. The monitor 205 in the present embodiment is a touch panel whereby the examiner can perform an input operation.

### <Measuring operation>

The operations of the apparatus configured as above will be explained below. In the ophthalmic apparatus 100 in the first embodiment, the control unit 200 sets any one of a corneal thickness measuring mode, an intraocular pressure measuring mode, and an iridocorneal-area photographing mode as a measuring mode of the ophthalmic apparatus 100. The iridocorneal-area photographing mode and the other modes may be differentiated by the control unit 200 in at least photographing control using the front observation optical system 30 and the cross-section imaging optical system 90, control of alignment means (e.g., the electrical moving mechanism 6) for the main unit 4 with respect to the examinee's eye E, display control of a monitor 80 for displaying the anterior-segment front image and the anterior-segment cross-sectional image, and analysis processing to obtain measurement parameters related to the anterior segment by analysis processing of the anterior-segment cross-sectional image. For instance, changing the display control may include not only changing an image of the examinee's eye E to be displayed but also changing an indication showing an active mode, and others. Further, changing the alignment control may include for example changing an alignment reference portion by automatic alignment, changing alignment guiding indication on a monitor, and others. Changing the analysis processing may include changing analysis algorism as well as changing an analysis target, the type of an analysis result, and others. One or some of the modes may be set not to perform the analysis processing.

The corneal thickness measuring mode is a mode of obtaining a still image of a cross-sectional image of a cornea including a corneal center of the examinee's eye E and then analyzing the obtained image to measure the corneal thickness of the examinee's eye E. The intraocular pressure measuring mode is a mode of placing the nozzle 7 in a predetermined positional relationship with the examinee's eye E and measuring the intraocular pressure of the examinee's eye E by the intraocular pressure measuring unit 10. The iridocorneal-area photographing mode is a mode of imaging the anterior-segment cross-sectional image including the iridocorneal area (hereinafter, an iridocorneal-area cross-sectional image) while the light projecting optical axis L1 is located at a point of iridocorneal area of the examinee's eye E.

In the first embodiment, each of the measuring modes may be arbitrarily selected by operation of the operating unit 201 by the examiner. Upon receipt of an operation signal representing the type of a measuring mode, the control unit 200 selects the measuring mode according to the operation signal. Measurements in each of the measuring modes may be executed in a predetermined order.

### <Corneal thickness measuring mode and Intraocular pressure measuring mode>

The first embodiment is explained below for convenience of explanation assuming that measurements are sequentially performed in the corneal thickness measuring mode and the intraocular pressure measuring mode (a continuous mode of the corneal thickness measuring mode and the intraocular pressure measuring mode), and then photographing in the iridocorneal-area photographing mode is carried out. In both the corneal thickness measuring mode and the intraocular pressure measuring mode, alignment of the main unit 4 with respect to the examinee's eye E is performed with reference to the corneal vertex (First alignment). Therefore, in the continuous mode including the corneal thickness measuring mode and the intraocular pressure measuring mode, alignment adjustment is carried out once, and then measurements are sequentially performed in each mode.

### <First alignment>

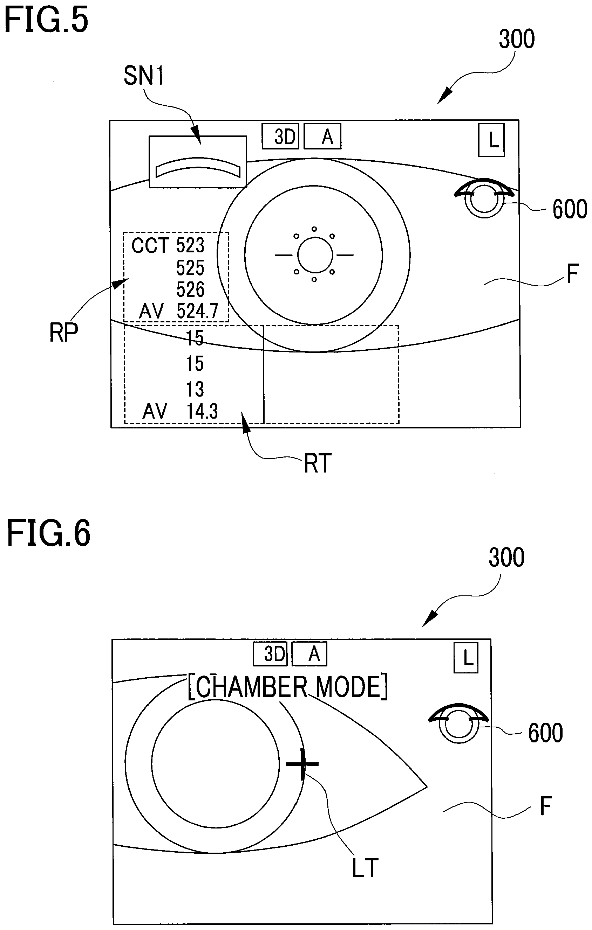

For alignment, the control unit 200 turns on the light source 41 for alignment in the X and Y directions, the fixation lamp 51, and the light source 61 for alignment in the Z direction. The control unit 200 starts obtaining and displaying a live image F (a moving image) of the anterior-segment front image. In other words, the control unit 200 turns on the light source 31 and also sequentially obtains the anterior-segment front image captured by the imaging element 37, and sequentially displays the obtained images on the monitor 205. Consequently, the live image F of the anterior-segment front image is displayed on an observation screen of the monitor 205 (see FIG. 5). In such a state, the examiner requests the examinee to fixate on the fixation target.

The first alignment may be achieved as automatic alignment. In this case, the control unit 200 controls the electrical moving mechanism 6 to form the luminescent spot by the light source 41 in an alignment position set on the imaging element 37 (e.g., at the intersection of the optical axis L1 and the imaging plane of the imaging element 37). Specifically, in both the corneal thickness measuring mode and the intraocular pressure measuring mode, photographing and measuring are performed while the light projecting optical axis L1 of the main unit 4 is placed at the corneal vertex. In an alignment completion position in the X and Y directions, specifically, when slit light is irradiated from the light projecting optical system 90a, the slit light will pass through the corneal vertex position.

In the first embodiment, after completion of the alignment in the X and Y directions, the alignment in the Z direction is performed. In the first embodiment, for the alignment adjustment in the Z direction, the control unit 200 controls the electrical moving mechanism 6 based on a working distance information obtained from the position detecting element 73 to move the main unit 4 toward a predetermined alignment completion position. As a result, in the first embodiment, the main unit 4 is moved in the Z direction so that the light projecting optical axis and the imaging optical axis of the cross-section imaging optical system 90 intersect at a place near the corneal vertex.

The first alignment may be performed by manually moving the main unit 4 by use of the joystick 5. Moreover, rough alignment may be conducted manually and fine adjustment may be performed by the foregoing automatic alignment.

### <Measurements of corneal thickness and intraocular pressure>

After completion of the first alignment, measurements of the corneal thickness and the intraocular pressure are performed. For instance, the control unit 200 starts the measurements based on a signal generated by operation of the measuring start switch 5b.

In the first embodiment, for convenience of explanation, the corneal thickness is first measured (the corneal thickness measuring mode).

After the relative position of the optical axis with respect to the corneal center of the examinee's eye E is adjusted by the electrical moving mechanism 6 and others so that the light section is formed at the corneal center of the anterior segment of the examinee's eye E, the control unit 200 obtains a still image of the cross-sectional image of the cornea of the examinee's eye E through the cross-section imaging optical system 90. The control unit 200 first turns on the light source 91 and captures the anterior-segment cross-sectional image based on a signal from the detector 97. As a result, a still image of the cross-sectional image including the corneal center is obtained. Thereafter, the control unit 200 obtains a measured value of the corneal thickness of the examinee's eye E by image analysis of the obtained cross-sectional image. The measured value of the corneal thickness and the cross-sectional image used for obtaining the measured value may be displayed on the monitor 205 by the control unit 200. One example of such a configuration is, as shown in FIG. 5, achieved by displaying the live image F of the anterior-segment front image together with a measured value RP and a corresponding cross-sectional image (a thumbnail SN (SN1) in FIG. 5). Of course, other configurations to be displayed may be adopted. The measured value and the corresponding cross-sectional image are stored in the memory 201.

Subsequently, the control unit 200 switches the measuring mode to the intraocular pressure measuring mode and measures the intraocular pressure of the examinee's eye E. In this case, the control unit 200 causes the intraocular pressure measuring unit 10 to blow compressed air to the cornea through the nozzle 7. The thus blown compressed air causes the cornea to be gradually deformed. At that time, the control unit 200 obtains an intraocular pressure value based on a deformation amount of the examinee's eye E detected by use of the photodetector 67. In the case of measuring the corneal thickness, the control unit 200 may correct the intraocular pressure value based on the corneal thickness of the examinee's eye E.

The intraocular pressure value may be displayed on the monitor 205 by the control unit 200. In the first embodiment, when measurement results (and imaging results) in a plurality of measuring modes are individually obtained, the measurement results in respective measuring modes can be displayed on the same screen. For example, when the measurement in the corneal thickness measuring mode and the intraocular pressure measuring mode is completed, a measurement value RP of the corneal thickness and a cross-sectional image SN of a corneal vertex portion corresponding to the measured value RP are displayed on the same screen in addition to the intraocular pressure value RT as shown in FIG. 5. The intraocular pressure value measured in the intraocular pressure measuring mode is stored in the memory 201.

### <Iridocorneal-angle photographing mode>

Switching from either the corneal thickness measuring mode or the intraocular pressure measuring mode to the iridocorneal-area photographing mode may be performed by operation of the operating unit 210 or may be automatically performed for example after completion of measurement of the corneal thickness or the intraocular pressure, and others.

In the first embodiment, when the iridocorneal-area photographing mode is set, the front observation optical system 30 irradiates visible light as illumination light to the examinee's eye E. In this case, the control unit 200 turns on the light source 38 to emit visible light as illumination light (the light source 31 may be turned off). This starts obtaining and displaying the anterior-segment front image by the visible light. Thus, the anterior-segment front image by the visible light displayed on the monitor 205 is made observable. Herein, the anterior-segment front image by the visible light has a clearer boundary between the cornea and the sclera as compared with the anterior-segment front image by infrared light. The iridocorneal area exists near the boundary and thus the position of the iridocorneal area on the anterior-segment front image may be specified by visual checking of the examiner (or by image analysis).

In the first embodiment, the main unit 4 is subjected to alignment (Second alignment) to adjust the point of iridocorneal area (the iridocorneal area position) of the examinee's eye E to a reference position of the alignment (namely, a target position at which the optical axis L1 is disposed), and then the anterior-segment cross-sectional image including the iridocorneal area (hereinafter, referred to as an iridocorneal-area cross-sectional image) is photographed (i.e., a still image is obtained). Adjustment of the relative position of the optical axis L1 with respect to the point of iridocorneal area of the examinee's eye E may be performed by an alignment mechanism (a driving mechanism) to move the main unit 4 with respect to the examinee's eye E. The alignment mechanism may be driven by manual operation of the examiner or may be driven automatically by the control unit 200. The alignment mechanism in the ophthalmic apparatus 100 is for example a moving mechanism provided in at least one of the table 1 and the movable base 3, and the electrical moving mechanism 6.

### <Second alignment>

In the first embodiment, the second alignment is performed manually by the examiner (Manual alignment). Specifically, the examiner makes an alignment operation with respect to the point of iridocorneal area while observing an image displayed on the monitor 205. During execution of the second alignment, a moving image (herein, a live image F) of the anterior-segment front image obtained by the front observation optical system 30 is displayed on an observation screen 300 of the monitor 205 (see FIG. 6).

In the first embodiment, the second alignment is made to adjust the position of the main unit 4 with respect to the examinee's eye E so that the optical axil L1 is set on the point of iridocorneal area located in a cutting direction of the slit light from the light projecting optical system 90a (that is, in a cutting direction of the light section in the first embodiment, more concretely in the X direction) with respect to the corneal vertex. Since the optical axis L1 is located at a predetermined position (e.g., the center of an image) of the front image in the first embodiment, the optical axis L1 is adjusted to coincide with the point of iridocorneal area. In this case, a reticle LT denoting the position of the light projecting optical axis L1 of the slit light may be electronically displayed on the anterior-segment front image displayed on the monitor 205.

For the alignment in the X and Y directions, it is preferably performed so that the optical axis L1 comes to a position closer to the corneal center of the examinee's eye E than a boundary position between the cornea and the sclera of the eye E (the details will be described later). In this case, in the X direction, the boundary between the cornea and the sclera of the anterior-segment front image serves as a target. In the Y direction, for example, the pupil center is a target. However, during the alignment in the X direction and the Y direction, the slit light may not be actually projected from the light projecting optical system 90a. The anterior-segment front image of visible light has a clearer boundary between the cornea and the sclera as compared with the anterior-segment front image of infrared light. Thus, the ophthalmic apparatus 100 can be appropriately operated for alignment in the X and Y directions. In this case, green light is preferably used as the visible light.

Herein, as described above, when the corneal thickness and the intraocular pressure are measured in advance, the examinee's eye E and the main unit 4 enter a positional relationship that allows the slit light from the light projecting optical system 90a to pass through the corneal vertex position as a result of the first alignment. In the iridocorneal-area photographing mode, the examiner adjusts the main unit 4 so that the optical axis L1 falls on the point of iridocorneal area. For instance, if the optical axis L1 coincides in advance with the corneal vertex, the examiner moves the main unit 4 (the movable base 3) linearly in either a right or left direction, thereby moving the optical axis L1 close to the iridocorneal area. This case allows the examiner to precisely and easily positon the optical axis L1 to the point of iridocorneal area located in the cutting direction of the slit light with respect to the corneal vertex.

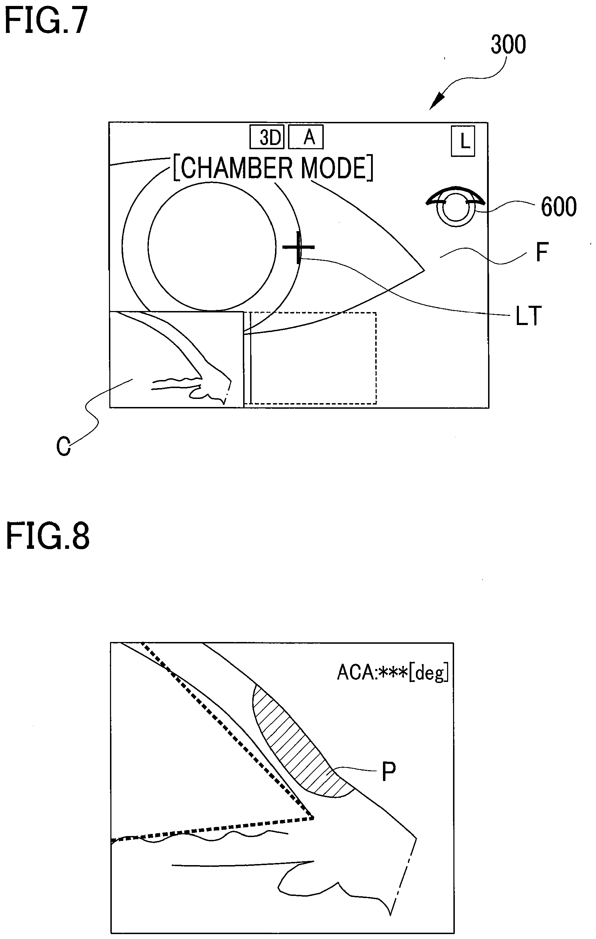

In the first embodiment, for example, when a button 600 on the observation screen 300 is selected by touching or clicking thereon, the control unit 200 starts obtaining and displaying a moving image (herein, a live image C) of a cross-sectional image of an iridocorneal area. In the first embodiment, for example, as shown in FIG. 7, the live image C of the cross-sectional image of the iridocorneal area is displayed together with a live image F of a front image of an anterior segment obtained by the front observation optical system 30. The control unit 200 turns on the light source 91 to project slit light to the anterior segment. Further, the control unit 200 sequentially obtains cross-sectional images of the iridocorneal area based on signals from the detector 97 and causes the monitor 205 to sequentially display the obtained cross-sectional images. Even though the slit light is visible light, the foregoing procedure starts projection of the slit light after an intersection of the projecting optical axis and the imaging optical axis of the cross-section imaging optical system 90 is roughly set near the point of iridocorneal area, so that most of the slit light will be eclipsed by iris and others and thus will not reach the fundus. Therefore, a burden on the examiner due to dazzling or the like is less likely to occur.

In the second alignment, the examiner adjusts the positional relationship of the main unit 4 in the Z direction with respect to the examinee's eye E so that the intersection of the projecting optical axis and the imaging optical axis of the cross-section imaging optical system 90 is set near the iridocorneal area. For instance, the examiner adjusts the main unit 4 to make both a corneal posterior surface and an iris anterior surface in the live image C of the iridocorneal-area cross-sectional image.

If an acute shape specific to the iridocorneal area does not appear in the live image C of the cross-sectional image of the iridocorneal area, the main unit 4 is further moved in the X and Y directions to make fine adjustment until the acute shape appears.

Herein, in a completion position of the first alignment, there is brought about such an alignment state in the Z direction in which the projecting optical axis and the imaging optical axis of the cross-section imaging optical system 90 intersect near the corneal vertex. The iridocorneal area is located at a position about 3 mm apart from the corneal vertex toward the fundus. When the iridocorneal-area photographing is to be performed in the iridocorneal-area photographing mode after the corneal thickness measuring mode (or the intraocular pressure measuring mode), for example, moving control may be performed to move the main unit 4 to a position near the anterior segment of the examinee's eye E in the Z direction with respect to the alignment completion position relative to the corneal vertex. As one example, in the present embodiment, the main unit 4 is moved in the Z direction by a predetermined distance (e.g., about 3 mm) (Offset movement). In this case, the control unit 200 in the present embodiment drives the electrical moving mechanism 6 to move the main unit 4 in the Z direction. This enables easy adjustment of the alignment in the Z direction. In the first embodiment, the examiner may further perform manual alignment after the Offset movement. This moving control may be executed for example when the control unit 200 receives an alignment completion signal (the details will be mentioned later) in the X and Y directions with respect to the iridocorneal area, when one mode is to be switched to the iridocorneal-area photographing mode, or at the time when one mode is switched to the iridocorneal-area photographing mode.

### <Photographing of cross-sectional image of iridocorneal area>

After completion of the second alignment, the cross-sectional image of the iridocorneal area is photographed. For instance, photographing may be performed by operation of the measurement start switch 5a of the joystick 5. By this photographing, the control unit 200 obtains a still image of the cross-sectional image of the iridocorneal area based on a signal from the detector 97. Further, the control unit 200 stores the obtained image in the memory 201. In the first embodiment, it is assumed that one-shot photographing is performed. Specifically, in one photographing, one still image is obtained and stored. In this case, the control unit 200 in the present embodiment causes the light source to emit slit light in a short time at a cycle of a frame rate of the anterior-segment cross-sectional image to be obtained, thereby photographing the still image. This can reduce image blurring due to movement of the examinee's eye E.

In the first embodiment, when the cross-sectional image of the iridocorneal area is to be photographed, the still image of the anterior-segment front image is photographed by the front observation optical system 30. In this case, the control unit 200 stores the still image of the anterior-segment front image obtained based on the signal from the imaging element 37, in association with the anterior-segment cross-sectional image, in the memory 201.

### <Display of photographing result and measurement result>

A still image of the cross-sectional image of the iridocorneal area photographed may be displayed, for example, in an enlarged size on the monitor 205 (see FIG. 8). For example, the control unit 200 may display information on the angle of the examinee's eye E (concretely, information on the angle of the anterior chamber, i.e., information on the angle of the acute shape specific to the iridocorneal angle area in the image) may be displayed together with the cross-sectional image of the iridocorneal area. The information on the angle may be for example a result obtained by visual measurement of the examiner by use of the cross-sectional image of the iridocorneal area or a result obtained by image analysis by the control unit 200. The information on the angle may be for example a numerical value representing the angle, an index emphasizing the acute shape on the cross-sectional image of the iridocorneal area (e.g., a V-shaped graphic pattern as shown in FIG. 8), or other information.

It is to be noted that a sign P represents a region in which luminance of the image is saturated. This region is likely to occur in a region of an anterior segment and its surrounding whereby scattering much light in an optical axis direction of the light receiving optical system 90b. It is important to reduce the region with saturated luminance in order to appropriately specify the shape of the iridocorneal area by visual observation on the cross-sectional image of the iridocorneal area or by image processing of the anterior-segment image. As described above, the photographing is performed while the optical axis L1 is located at a position closer to the corneal center of the examinee's eye E than the boundary position between the cornea and the sclera of the examinee's eye E. This can suppress the region with saturated luminance and also obtain an image allowing easy observation of an acute shape specific to the iridocorneal area.

The iridocorneal-area cross-sectional image and the anterior-segment front image photographed in association with the iridocorneal-area cross-sectional image may be displayed on the monitor 205. In this case, these two images may be displayed at the same time or selectively displayed one by one. In those cases, for example, the anterior-segment front image may be displayed with an index denoting the photographing position of the iridocorneal-area cross-sectional image (the irradiation position of the slit light) in a superimposing manner. In the first embodiment, for instance, since the position of the projecting optical axis of the slit light in the anterior-segment front image is fixed, the index may be displayed by superimposing at that fixed position. This enables the examiner for example to easily ascertain the photographing position of the cross-sectional image of the iridocorneal area in the anterior segment.

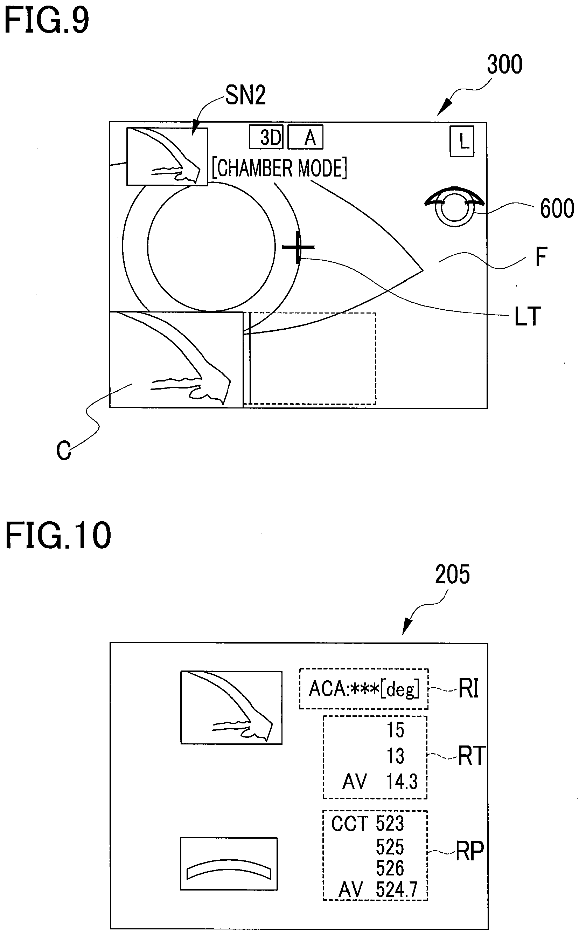

Further, the control unit 200 may display a thumbnail SN2 of the cross-sectional image of the iridocorneal area together with the live image F of the anterior-segment front image (see FIG. 9). In this case, for thumbnail display, for example, the control unit 200 detects the position of an outline of a cross-sectional image included in the captured image by the imaging element 97, and sets a display region for displaying the thumbnail on the monitor 205 with respect to the captured image based on the detected outline position. The control unit 200 cuts out image data corresponding to the set display region and displays the thumbnail SN2 of the cross-sectional image on the monitor 205. As a result, even a cross-sectional image obtained when the main unit 4 is misaligned with respect to the examinee's eye E can be displayed in the form of a thumbnail. Further, the iridocorneal-area cross-sectional image displayed as the thumbnail may be displayed together with the information on the angle of the acute shape specific to the iridocorneal area in the image.

As shown in FIG. 10, the cross-sectional image of the iridocorneal area may be displayed together with other measurement results and photographing results obtained by the ophthalmic apparatus 100. In the example shown in FIG. 10, for example, a still image of the cross-sectional image of the iridocorneal area is displayed side by side with the information on the angle of the anterior chamber (e.g., an angle RI in FIG. 10) and also with a measured value RT of intraocular pressure, a measured value RP of corneal thickness, and a corneal cross-sectional image including the corneal center. It is to be noted that those images and information (measured values, analysis results, and others) are not necessarily displayed on the same screen, and may be displayed on separate screens. The images and the measurement results obtained in each mode may also be displayed on the observation screen 300 as shown in FIG. 9.

The cross-sectional image of the iridocorneal area, the angle RI, and the intraocular pressure value are useful indices of glaucoma. Accordingly, displaying them together enables the examiner to appropriately perform a test (e.g., screening) for glaucoma. It is to be noted that only one of the cross-sectional image of the iridocorneal area and the information on the angle of the anterior chamber may be displayed together with the intraocular pressure value.

Further, the cross-sectional image of the iridocorneal area may also be displayed on the monitor 205 together with a measured value of corneal thickness and a cross-sectional image (so-called a pachymetry image) utilized for calculation of the measured value.

As explained above, the ophthalmic apparatus 100 can obtain the cross-sectional image of the iridocorneal area through a simple structure.

In particular, the ophthalmic apparatus 100 photographs the cross-sectional image of the iridocorneal area while the projecting optical axis L1 is set on the iridocorneal area, so that a good cross-sectional image of the iridocorneal area is likely to be obtained.

The ophthalmic apparatus 100 can obtain the cross-sectional image including the iridocorneal area and the cross-sectional image including the corneal center through a simple structure.

According to the ophthalmic apparatus 100 in the first embodiment, a single apparatus can carry out iridocorneal-area photographing and intraocular pressure measurement, thus effectively performing diagnosis of the examinee's eye (e.g., diagnosis of glaucoma).

### <Second Embodiment>

A second embodiment of this disclosure will be explained below. The ophthalmic apparatus 100 in the second embodiment is configured to perform the second alignment in the iridocorneal-area photographing mode by automatic alignment based on moving control of the main unit 4 by the control unit 200. The apparatus structure of the ophthalmic apparatus 100 in the second embodiment is identical to that in the first embodiment if not otherwise specified. The following explanation is given to the second alignment in the second embodiment.

In the second embodiment, an alignment state with respect to the point of iridocorneal area is detected by the control unit 200 based on image information on the iridocorneal area. For the image information, the anterior-segment front image obtained by the front observation optical system 30 may be utilized or the cross-sectional image obtained by the cross-section imaging optical system 90 may be utilized. For instance, at least an alignment state in up-and-down and right-and-left directions may be detected based on the anterior-segment front image. Further, at least an alignment state in the working distance may be detected based on the anterior-segment cross-sectional image.

In the second embodiment, for example, the alignment state with respect to the point of iridocorneal area is detected using the anterior-segment front image. For instance, the control unit 200 detects the point of iridocorneal area from the live image F of the anterior-segment front image obtained from the imaging element 37 of the front observation optical system 30. For example, the point of iridocorneal area is detected based on a boundary between the cornea and the sclera (a boundary between black and white) in the anterior-segment front image. In this case, for example, the boundary may be detected by image processing such as edge detection. As an alternative, a boundary between iris and sclera may be detected.

In the second embodiment, the control unit 200 detects the point of iridocorneal area located on a cutting line of slit light with respect to the corneal vertex. This point of iridocorneal area exists one in each of right and left sides on a horizontal line passing through the corneal vertex. Either one of the right and left point of iridocorneal areas to be detected by the control unit 200 may be for example selected by the control unit 200 according to a command from an examiner or determined in advance. The control unit 200 detects the alignment state of the main unit 4 with respect to the examinee's eye E based on a positional relationship between the point of iridocorneal area in the anterior-segment front image and the projecting optical axis L1.

The control unit 200 drives the electrical moving mechanism 6 to move the main unit 4 so that the optical axis L1 establishes a predetermined positional relationship with the detected point of iridocorneal area. When the alignment state is detected in which the point of iridocorneal area and the optical axis L1 establish the predetermined positional relationship, the alignment in the X and Y directions is completed. In this case, a target position in the X direction in the present embodiment may be set for example with reference to the boundary between the cornea and the sclera in the anterior-segment front image. In the Y direction, for example, the pupil center may be set as a reference.

Even for the alignment in the X and Y directions in the second embodiment, the anterior-segment front image formed by the visible light may be used as in the first embodiment. Since the anterior-segment front image by the visible light has a clear boundary between the cornea and the sclera, serving as a target to the point of iridocorneal area, the alignment state can be appropriately detected. This enables precise alignment in the X and Y directions.

After completion of the alignment in the X and Y directions, the alignment in the Z direction is performed. This Z-direction alignment is performed using the anterior-segment cross-sectional image (e.g., the iridocorneal-area cross-sectional image). At this time, the offset movement in the Z direction explained in the first embodiment may also be executed together. When the Z-direction alignment is to be performed, the control unit 200 obtains a live image C of the anterior-segment cross-sectional image. The control unit 200 then analyzes the anterior-segment cross-sectional image (e.g., the iridocorneal-area cross-sectional image) to detect the Z-direction alignment state. For this Z-direction alignment state, for example, a portion such as the cornea and the iris may be detected by analysis of the cross-sectional image so that the Z-direction alignment is detected based on a position of the portion in the image.

Based on a detection result of the Z-direction alignment state, the control unit 200 drives the electrical moving mechanism 6 to move the main unit 4 in the Z direction. As a result, the alignment position in the Z-direction is adjusted so that the intersection of the projecting optical axis and the imaging optical axis of the cross-section imaging optical system 90 is set near the iridocorneal area.

In this case, detailed alignment in the X and Y directions may be conducted in such a way of analyzing the cross-sectional image and detecting displacement of the analyzed cross-sectional image from an image obtained when the acute shape of the iridocorneal area is included in a predetermined position of the cross-sectional image, and driving the electrical moving mechanism 6 based on a displacement amount. In this way, the second alignment may be automatically performed.

### <Modified Example>

The technique of this disclosure is explained based on the foregoing embodiments but is not necessarily limited to the foregoing embodiments.

For instance, in the above-described first embodiment, the second alignment is performed manually by an examiner who grasps the alignment state from the live image (either the front image or the cross-sectional image) of the anterior segment displayed on the monitor 80. As an alternative, for example, when the second alignment is to be manually performed, the control for detecting the alignment state as shown in the second embodiment may be performed. In this case, for example, the control unit may display an alignment guide for directing the optical axis toward the iridocorneal area based on an alignment detection result obtained based on either the front image or the cross-sectional image of the anterior segment. The signal representing alignment completion in the X and Y directions may be a signal commanding the start of obtaining a live image C of the iridocorneal-area cross-sectional image. In the present example, for example, the moving control may be started upon selection of the button 600.

In the foregoing embodiments, one still image of the anterior-segment cross-sectional image is obtained in one photographing. As an alternative, a plurality of still images of the anterior-segment cross-sectional image may be obtained sequentially in one photographing. This enables easily obtaining a good anterior-segment cross-sectional image by one photographing, even when misalignment of the main unit 4 from the examinee's eye E is present during photographing. Further, after photographing, the control unit 200 may select at least one image to be utilized preferentially to other images from among a plurality of anterior-segment cross-sectional images sequentially photographed. The image(s) to be preferentially used may be automatically selected based on an analysis result of each image. The image(s) to be preferentially used may also be manually selected by operation of the examiner to designate the image(s) to be preferentially used. In this case, for example, the control unit 200 displays the sequentially photographed images side by side on the monitor 205. The image(s) to be preferentially used is selected based on a signal input to the control unit 200 through the operating unit 210 operated by the examiner. In this case, any images other than the selected images by the control unit 200 may be deleted from the memory 201. As still another alternative, each image may be assigned a preference order and the images in a data set of the images obtained in one photographing based on the preference order may be sorted.

To reduce the region P with saturated luminance as shown in FIG. 8, moreover, the control unit 200 may adjust either one of the light quantity of the light source 91 or the gain of the detector 97 and photograph the iridocorneal-area cross-sectional image. Adjusting the light quantity and the gain may be performed manually by operation of the examiner or automatically by the control unit 200. As one example of automatic adjustment, there is a case where either the light quantity or the gain is adjusted based on a result of image analysis on the iridocorneal-area cross-sectional image. For example, when the anterior-segment image obtained as the live image C contains the pixels with saturated luminance more than a predetermined ratio, the control unit 200 may perform such a control on the light source 91 as to reduce either the light quantity or the gain. For instance, this control may be performed when the control unit 200 detects an acute shape specific to the iridocorneal area (more concretely, an acute shape defined by a corneal posterior surface and an iris anterior surface) by analysis of the iridocorneal-area cross-sectional image. When pixels with sufficient luminance are not enough, the control of increasing the light quantity or the gain of the cross-section imaging optical system 90 may be performed. When either the light quantity or the gain is adjusted as above, the ophthalmic apparatus 100 can appropriately photograph the iridocorneal-area cross-sectional image.

The alignment state with respect to the point of iridocorneal area can be detected by use of the anterior-segment front image as described above. In this case, for example, the anterior-segment front image obtained as the live image is analyzed to detect either the cornea or the iris.

To reduce a region with saturated luminance, an exposure time of the photodetector 97 may be controlled. For instance, the control unit 200 may adjust the exposure time during photographing based on an analysis result on the luminance of the iridocorneal-area cross-sectional image obtained in advance.

In the second embodiment, the automatic alignment in the Z direction is performed on the anterior-segment cross-sectional image. However, this disclosure is not limited thereto and may be configured to perform only the offset movement in the Z direction as explained in the first embodiment.

In the foregoing second embodiment, the second alignment uses automatic alignment by the control unit 200 for both the alignment in the X and Y directions and the alignment in the Z direction. As an alternative, manual alignment may be partially used. For instance, it may be arranged such that the automatic alignment is used for one of the alignment in the X and Y directions and the alignment in the Z direction and the manual alignment is used for the other.

As explained in each of the foregoing embodiments, the control unit 200 may execute, in the iridocorneal-area photographing mode, the moving control to adjust the position of the main unit 4 (and the cross-section imaging optical system 90) to a position nearer the anterior segment in the Z direction than a position at which the still image of the cross-sectional image of the cornea is obtained in the corneal thickness measuring mode. In each of the foregoing embodiments, this moving control is not limited to for moving the main unit 4 by a predetermined distance. For instance, the control unit 200 may move forward an optometry part based on the signal representing the alignment completion in the X and Y directions and, upon detection of the cross-sectional image of the iridocorneal area, automatically stop the forward movement of the optometry part. Further, the control unit 200 may automatically move the main unit 4 so as to form the cross-sectional image of the iridocorneal area at a predetermined position. In this case, the alignment completion signal serving as a trigger for forward movement may be output at a stage in which the optical axis L1 is placed at a position displaced to some extent from the alignment completion position in the X and Y directions (e.g., a position set to perform photographing the cross-sectional image of the iridocorneal area in the X and Y directions).

In the iridocorneal-area photographing mode, when a predetermined time has elapsed from the start of projecting the slit light (namely, light projection by the cross-section imaging optical system 90) to obtain the live image of the anterior-segment cross-sectional image, the control unit 200 may automatically stop light projection. This can suppress the slit light from placing an excessive burden on the examinee's eye E.

In the foregoing embodiments, the slit light from the light projecting optical system 90a is irradiated to the examinee's eye E from front. However, this disclosure is not limited thereto. The slit light may also be irradiated to the examinee's eye E from an oblique direction. For instance, the light projecting optical system 90a may be configured to project the slit light from the outside of the nozzle 7. For one example thereof, refer to an optical system disclosed in JP-A-2008-011878.

In the foregoing embodiments, the ophthalmic apparatus is exemplified by the apparatus provided with the optical systems based on the Scheimpflug's principle and configured to photograph the cross-sectional image of the iridocorneal area, but this disclosure is not limited thereto. For instance, this disclosure may be applied to any anterior-segment measuring apparatus including a light projecting optical system for projecting light emitted from a light source toward an anterior segment of an examinee's eye to form a light section on the anterior segment and a light receiving optical system including a detector to receive anterior-segment scattered light obtained by scattering of the light section at the anterior segment, and being configured to form the anterior-segment cross-sectional image based on a detection signal from the detector to measure the anterior segment by processing of the cross-sectional image. For instance, this disclosure may be applied to an anterior-segment tomographic image photographing apparatus (Optical Coherence Tomography: OCT) including an interference optical system for causing a light receiving element to receive interference light obtained by synthesis of measurement light reflected by the examinee's eye and reference light, the apparatus being configured to photograph a tomographic image of the anterior segment of the examinee's eye.

In the anterior-segment OCT, for example, an optical scanner scans a measurement beam over the anterior segment. In this case, there is provided a sensor for detecting positional displacement in a vertical direction from an imaging plane of a tomographic image (in a scanning direction of the measurement light) (e.g., a CCD camera for obtaining a front image of an anterior segment, an SLO (Scanning laser ophthalmoscope), an OCT front image obtained by two-dimensional scanning of OCT, and others), so that displacement correcting processing is performed based on an output signal from the sensor.

### Explanation of Reference signs

- 1: Table
- 3: Movable base
- 4: Main unit
- 6: Electrical moving mechanism
- 200: Control unit
- 201: Memory
- 30: Front observation optical system
- 40: Alignment optical system
- 90: Cross-section imaging optical system
- 205: Monitor
- 90a: Light projecting optical system
- 90b: Light receiving optical system
- 91: Light source
- 97: Detector
- L1: Optical axis

## Claims

1. An ophthalmic apparatus comprising:
a front observation optical system (30) for observing a front image of an anterior segment of an examinee's eye (E);
a cross-section imaging optical system (90) including a light projecting optical system (90a) for projecting slit light toward the anterior segment of the examinee's eye to form a light section on the anterior segment so that the light section passes through an optical axis (L1) of the front observation optical system, and a light receiving optical system (90b) for receiving anterior-segment scattered light obtained by scattering of the light section at the anterior segment, the cross-section imaging optical system (90) being arranged to capture a cross-sectional image of the anterior segment of the examinee's eye (E);
iridocorneal area photographing means (200) configured for adjusting a relative position of the optical axis with respect to an iridocorneal area of the examinee's eye so that the light section is formed on the iridocorneal area of the examinee's eye, and obtaining a still image of a cross-sectional image of the iridocorneal area through the cross-section imaging optical system,
wherein the iridocorneal-area photographing means is configured to make alignment of the optical axis with respect to the iridocorneal area of the examinee's eye to form a light section on the iridocorneal area of the examinee's eye and obtain the still image of the cross-sectional image of the iridocorneal area of the examinee's eye by the cross-section imaging optical system; and
a cornea photographing means (200) for adjusting a relative position of the optical axis with respect to a corneal center of the anterior segment of the examinee's eye so that the light section is formed on the corneal center of the examinee's eye, and obtaining a still image of a cross-sectional image of the cornea through the cross-section imaging optical system,
**characterized in that** the ophthalmic apparatus is further comprising:
an intraocular pressure measuring unit (10) for measuring intraocular pressure of the examinee's eye (E); and
a mode setting means for setting either a first mode of photographing a cornea and measuring a corneal thickness of the examinee's eye (E), or a second mode of measuring the intraocular pressure of the examinee's eye (E) by the intraocular pressure measuring unit (10), or a third mode for photographing an iridocorneal area.

2. The ophthalmic apparatus according to claim 1, comprising:
first display control means (200) serving as display control means for displaying, on a display part (205), an intraocular pressure value measured by the intraocular pressure measuring unit and the still image of the cross-sectional image of the iridocorneal area of the examinee's eye photographed by the iridocorneal-area photographing means.

3. The ophthalmic apparatus according to claim 1 or 2, further comprising:
control means (200) for changing control according to the photographing mode set by the mode setting means,
wherein the control means is at least one of:
(a) control means for performing photographing control of the front observation optical system (30) and the cross-section imaging optical system (90), the control means (a) being configured to change the photographing control according to the mode set by the mode setting means;
(b) control means for performing alignment control of an optometry unit (4) provided with the front observation optical system and the cross-section imaging optical system with respect to the examinee's eye, the control means (b) being configured to change the alignment control according to the mode set by the mode setting means;
(c) control means for performing control of the display unit (205) for displaying the anterior-segment front image and the anterior-segment cross-sectional image, the control means (c) being configured to change the display control according to the mode set by the mode setting means;
(d) control means for performing analysis processing the anterior-segment cross-sectional image to obtain a measurement parameter related to the anterior segment, the control means (d) being configured to switch the analysis processing according to the mode set by the mode setting means; and
(e) control means for storing the anterior-segment cross-sectional image in a storage unit (201), the control means (e) being configured to store a photographing portion corresponding to the mode set by the mode setting means, in association with the anterior-segment cross-sectional image.

4. The ophthalmic apparatus according to any one of claims 1 to 3, wherein the iridocorneal-area photographing means includes alignment detecting means for detecting an alignment state of the optical axis with respect to the iridocorneal area of the examinee's eye.

5. The ophthalmic apparatus according to claim 4, further comprising automatic alignment means for adjusting a relative position of the optical axis with respect to the iridocorneal area based on a detection result of the alignment detecting means.

6. The ophthalmic apparatus according to any one of claims 1 to 5, comprising:
anterior-segment front imaging means for obtaining a still image of an anterior-segment front image by the front image observing optical system when the cross-sectional image of the iridocorneal area of the examinee's eye is to be photographed; and
second display control means serving as display control means for displaying, on the display unit, the cross-sectional image of the iridocorneal area photographed by the iridocorneal-area photographing means and the anterior-segment front image photographed by the anterior-segment front photographing means.

7. The ophthalmic apparatus according to any one of claims 1 to 6, wherein
the iridocorneal-area photographing means is configured to adjust a position of the cross-section imaging optical system to a position closer to the anterior segment of the examinee's eye relative to a working distance direction than a position at which the still image of the cross-sectional image of the cornea of the examinee's eye by the cornea photographing means.

8. The ophthalmic apparatus according to any one of claims 1 to 7, wherein
the iridocorneal-area photographing means is configured to adjust the relative position so that the optical axis is disposed at a position in which the light section is formed on the iridocorneal area of the examinee's eye, the position being closer to a corneal center of the examinee's eye than a boundary position between a cornea and a sclera of the examinee's eye.

9. The ophthalmic apparatus according to any one of claims 1 to 8, wherein
the front observation optical system includes a visible light source (38),
when the iridocorneal-area photographing mode is set by the mode setting means, the visible light source is turned on to enable observation of a visible front image through the front observation optical system.

10. The ophthalmic apparatus according to any one of claims 1 to 9, wherein
the light receiving optical system includes optical arrangement placed obliquely with respect to a light projecting optical axis of the light projecting optical system and based on a Scheimpflug's relationship.

11. The ophthalmic apparatus according to any one of claims 1 to 10, further comprising display control means for displaying, on the display unit, information on the angle of the examinee's eye together with the still image of the cross-sectional image of the iridocorneal area of the examinee's eye photographed by the iridocorneal-area photographing means.

12. The ophthalmic apparatus according to any one of claims 1 to 11, further comprising a fixation target optical system for fixing a visual line of the examinee's eye frontward,
wherein the iridocorneal-area photographing means is configured to obtain the still image of the cross-sectional image of the iridocorneal area of the examinee's eye by the cross-section imaging optical system with respect to the examinee's eye fixed frontward by the fixation target projecting optical system.

## Patentansprüche

1. Eine ophthalmische Vorrichtung, enthaltend
ein optisches Frontalbeobachtungssystem (30) zum Beobachten eines Frontalbildes eines anterioren Segments des Auges (E) eines zu Untersuchenden;
ein optisches Querschnittsbilderfassungssystem (90), enthaltend ein optisches Lichtprojektionssystem (90a) zum Projizieren von Spaltlicht auf das anteriore Segment des Auges des zu Untersuchenden, um einen Lichtschnitt auf dem anterioren Segment so zu bilden, dass der Lichtschnitt durch eine optische Achse (L1) des optischen Frontalbeobachtungssystems verläuft, und ein optisches Lichtempfangssystem (90b) zum Empfangen von Streulicht des anterioren Segments, das durch Streuung des Lichtschnitts an dem anterioren Segment erhalten wird, wobei das optische Querschnittsbilderfassungssystem (90) so angeordnet ist, dass es ein Querschnittsbild des anterioren Segments des Auges des zu Untersuchenden (E) erfasst;
eine Einrichtung (200) zum Fotografieren des iridokornealen Bereichs, die dazu konfiguriert ist, eine relative Position der optischen Achse in Bezug auf einen iridokornealen Bereich des Auges des zu Untersuchenden so einzustellen, dass der Lichtschnitt auf dem iridokornealen Bereich des Auges des zu Untersuchenden gebildet wird, und ein Standbild eines Querschnittsbildes des iridokornealen Bereichs durch das optische Querschnittsbilderfassungssystem zu erhalten,
wobei die Einrichtung zum Fotografieren des iridokornealen Bereichs dazu konfiguriert ist, eine Ausrichtung der optischen Achse in Bezug auf den iridokornealen Bereich des Auges des zu Untersuchenden vorzunehmen zum Bilden eines Lichtschnitts auf dem iridokornealen Bereich des Auges des zu Untersuchenden und das Standbild des Querschnittsbildes des iridokornealen Bereichs des Auges des zu Untersuchenden durch das optische Querschnittsbilderfassungssystem zu erhalten; und
eine Hornhautfotografiereinrichtung (200) zum Einstellen einer relativen Position der optischen Achse in Bezug auf ein Hornhautzentrum des anterioren Segments des Auges des zu Untersuchenden, so dass der Lichtschnitt auf dem Hornhautzentrum des Auges des zu Untersuchenden gebildet wird, und zum Erhalten eines Standbildes eines Querschnittsbildes der Hornhaut durch das optische Querschnittsbilderfassungssystem,
**dadurch gekennzeichnet, dass** die ophthalmische Vorrichtung ferner enthält:
eine Augeninnendruck-Messeinheit (10) zum Messen des Augeninnendrucks des Auges (E) des zu Untersuchenden; und
eine Moduseinstelleinrichtung zum Einstellen entweder eines ersten Modus des Fotografierens einer Hornhaut und des Messens einer Hornhautdicke des Auges (E) des zu Untersuchenden oder eines zweiten Modus des Messens des Augeninnendrucks des Auges (E) des zu Untersuchenden durch die Augeninnendruckmesseinheit (10) oder eines dritten Modus des Fotografierens eines iridokornealen Bereichs.

2. Die ophthalmische Vorrichtung gemäß Anspruch 1, enthaltend
eine erste Anzeigesteuereinrichtung, (200), die als Anzeigesteuereinrichtung zum Anzeigen eines von der Augeninnendruckmesseinheit gemessenen Augeninnendruckwerts und des Standbilds des Querschnittsbildes des von der Einrichtung zum Fotografieren des iridokornealen Bereichs fotografierten iridokornealen Bereichs des Auges des zu Untersuchenden auf einer Anzeigeeinheit (205) dient.

3. Die ophthalmische Vorrichtung gemäß Anspruch 1 oder 2, ferner enthaltend:
eine Steuereinrichtung (200) zum Ändern der Steuerung entsprechend dem durch die Moduseinstelleinrichtung eingestellten Fotografiermodus,
wobei die Steuereinrichtung zumindest eine ist aus:
(a) einer Steuereinrichtung zum Durchführen einer Fotografiesteuerung des optischen Frontalbeobachtungssystems (30) und des optischen Querschnittsbilderfassungssystems (90), wobei die Steuereinrichtung (a) dazu konfiguriert ist, die Fotografiesteuerung gemäß dem durch die Moduseinstelleinrichtung eingestellten Modus zu ändern;
(b) einer Steuereinrichtung zum Durchführen einer Ausrichtungssteuerung einer Optometrieeinheit (4), die mit dem optischen Frontalbeobachtungssystem und dem optischen Querschnittsbilderfassungssystem bereitgestellt ist, in Bezug auf das Auge des zu Untersuchenden, wobei die Steuereinrichtung (b) dazu konfiguriert ist, die Ausrichtungssteuerung gemäß dem durch die Moduseinstelleinrichtung eingestellten Modus zu ändern;
(c) einer Steuereinrichtung zum Durchführen einer Steuerung der Anzeigeeinheit (205) zum Anzeigen des Frontalbildes des anterioren Segments und des Querschnittsbildes des anterioren Segments, wobei die Steuereinrichtung (c) dazu konfiguriert ist, sie die Anzeigesteuerung entsprechend dem durch die Moduseinstelleinrichtung eingestellten Modus zu ändern;
(d) einer Steuereinrichtung zum Durchführen einer Analyseverarbeitung des Querschnittsbildes des anterioren Segments, um einen auf das anteriore Segment bezogenen Messparameter zu erhalten, wobei die Steuereinrichtung (d) dazu konfiguriert ist, die Analyseverarbeitung gemäß dem durch die Moduseinstelleinrichtung eingestellten Modus zu schalten; und
(e) einer Steuereinrichtung zum Speichern des Querschnittsbildes des anterioren Segments in einer Speichereinheit (201), wobei die Steuereinrichtung (e) dazu konfiguriert ist, einen Fotografierabschnitt, der dem durch die Moduseinstelleinrichtung eingestellten Modus entspricht, in Verbindung mit dem Querschnittsbild des anterioren Segments zu speichern.

4. Die ophthalmische Vorrichtung gemäß einem der Ansprüche 1 bis 3, wobei die Einrichtung zum Fotografieren des iridokornealen Bereichs eine Ausrichtungserfassungseinrichtung zum Erfassen eines Ausrichtungszustands der optischen Achse in Bezug auf den iridokornealen Bereich des Auges des zu Untersuchenden umfasst.

5. Die ophthalmische Vorrichtung gemäß Anspruch 4, ferner enthaltend eine automatische Ausrichtungseinrichtung zum Einstellen einer relativen Position der optischen Achse in Bezug auf den iridokornealen Bereich auf der Grundlage eines Erfassungsergebnisses der Ausrichtungserfassungseinrichtung.

6. Die ophthalmische Vorrichtung gemäß einem der Ansprüche 1 bis 5, enthaltend:
eine Frontalbilderfassungseinrichtung für das anteriore Segment zum Erhalten eines Standbilds des Frontalbildes des anterioren Segments durch das optische Frontalbildbeobachtungssystem, wenn das Querschnittsbild des iridokornealen Bereichs des Auges des zu Untersuchenden fotografiert werden soll; und
eine zweite Anzeigesteuereinrichtung, die als Anzeigesteuereinrichtung zum Anzeigen des Querschnittsbildes des iridokornealen Bereichs, das durch die Einrichtung zum Fotografieren des iridokornealen Bereichs fotografiert wurde, und des Frontalbildes des anterioren Segments, das durch die Frontalfotografiereinrichtung des anterioren Segments fotografiert wurde, auf der Anzeigeeinheit dient.

7. Die ophthalmische Vorrichtung gemäß einem der Ansprüche 1 bis 6, wobei die Einrichtung zum Fotografieren des iridokornealen Bereichs dazu konfiguriert ist, eine Position des optischen Querschnittsbilderfassungssystems auf eine Position einzustellen, die relativ zu einer Arbeitsabstandsrichtung näher an dem anterioren Segment des Auges des zu Untersuchenden liegt als eine Position, an der das Standbild des Querschnittsbildes der Hornhaut des Auges des zu Untersuchenden durch die Hornhautfotografiereinrichtung aufgenommen wird.

8. Die ophthalmische Vorrichtung gemäß einem der Ansprüche 1 bis 7, wobei die Einrichtung zum Fotografieren des iridokornealen Bereichs dazu konfiguriert ist, die relative Position so einzustellen, dass die optische Achse an einer Position angeordnet ist, in der der Lichtschnitt auf dem iridokornealen Bereich des Auges des zu Untersuchenden gebildet wird, wobei die Position näher an einem Hornhautzentrum des Auges des zu Untersuchenden liegt als eine Grenzposition zwischen einer Hornhaut und einer Sklera des Auges des zu Untersuchenden.

9. Die ophthalmische Vorrichtung gemäß einem der Ansprüche 1 bis 8, wobei
das optische Frontalbeobachtungssystem eine sichtbare Lichtquelle (38) enthält,
die sichtbare Lichtquelle eingeschaltet wird, wenn der Fotografiermodus für den iridokornealen Bereich durch die Moduseinstelleinrichtung eingestellt wird, um die Beobachtung eines sichtbaren Frontalbildes durch das optische Frontalbeobachtungssystem zu ermöglichen.

10. Die ophthalmische Vorrichtung gemäß einem der Ansprüche 1 bis 9, wobei das optische Lichtempfangssystem eine optische Anordnung umfasst, die schräg in Bezug auf eine optische Lichtprojektionsachse des optischen Lichtprojektionssystems angeordnet ist, und auf einer Scheimpflug-Beziehung basiert.

11. Die ophthalmische Vorrichtung gemäß einem der Ansprüche 1 bis 10, ferner enthaltend eine Anzeigesteuereinrichtung, um auf der Anzeigeeinheit Informationen über den Winkel des Auges des zu Untersuchenden zusammen mit dem von der Einrichtung zum Fotografieren des iridokornealen Bereichs aufgenommenen Standbild des Querschnittsbildes des iridokornealen Bereichs des Auges des zu Untersuchenden anzuzeigen.

12. Die ophthalmische Vorrichtung gemäß einem der Ansprüche 1 bis 11, ferner enthaltend ein optisches Fixierungszielsystem zum Fixieren einer visuellen Linie des Auges des zu Untersuchenden nach vorne,
wobei die Einrichtung zum Fotografieren des iridokornealen Bereichs dazu konfiguriert ist, das Standbild des Querschnittsbildes des iridokornealen Bereichs des Auges des zu Untersuchenden durch das optische Querschnittsbilderfassungssystem in Bezug auf das Auge des zu Untersuchenden zu erhalten, das durch das optische Fixierungszielprojektionssystem nach vorne fixiert ist.

## Revendications

1. Un appareil ophtalmique comprenant
un système optique d'observation frontale (30) pour observer une image frontale d'un segment antérieur de l'œil d'un examiné (E) ;
un système optique d'imagerie en coupe transversale (90) comprenant un système optique de projection de lumière (90a) pour projeter une lumière en fente vers le segment antérieur de l'œil de l'examiné pour former une section de lumière sur le segment antérieur de sorte que la section de lumière passe par un axe optique (L1) du système optique d'observation frontale, et un système optique de réception de la lumière (90b) pour recevoir la lumière diffusée du segment antérieur obtenue par diffusion de la section de lumière au niveau du segment antérieur, le système optique d'imagerie en coupe transversale (90) étant disposé pour capturer une image en coupe transversale du segment antérieur de l'œil de l'examiné (E) ;
un moyen photographie de la zone iridocornéenne (200) configurés pour ajuster une position relative de l'axe optique par rapport à une zone iridocornéenne de l'œil de l'examiné de sorte que la section de lumière soit formée sur la zone iridocornéenne de l'oeil de l'examiné, et pour obtenir une image fixe d'une image en coupe transversale de la zone iridocornéenne par l'intermédiaire du système optique d'imagerie en coupe transversale,
dans lequel le moyen de photographie de la zone iridocornéenne est configuré pour réaliser l'alignement de l'axe optique par rapport à la zone iridocornéenne de l'oeil de l'examiné afin de former une section de lumière sur la zone iridocornéenne de l'oeil de l'examiné et d'obtenir l'image fixe de l'image en coupe transversale de la zone iridocornéenne de l'œil de l'examiné par le système optique d'imagerie en coupe transversale ; et
un moyen de photographie de la cornée (200) pour ajuster une position relative de l'axe optique par rapport à un centre cornéen du segment antérieur de l'œil de l'examiné de sorte que la section de lumière soit formée sur le centre cornéen de l'œil de l'examiné, et pour obtenir une image fixe d'une image en coupe transversale de la cornée par le système optique d'imagerie en coupe transversale,
**caractérisé en ce que** l'appareil ophtalmique comprend en outre :
une unité de mesure de la pression intraoculaire (10) pour mesurer la pression intraoculaire de l'œil de l'examiné (E) ; et
un moyen de réglage de mode pour établir soit un premier mode de photographie d'une cornée et de mesure d'une épaisseur cornéenne de l'œil (E) de l'examiné, soit un second mode de mesure de la pression intraoculaire de l'œil (E) de l'examiné par l'unité de mesure de la pression intraoculaire (10), soit un troisième mode de photographie d'une aire iridocornéenne.

2. L'appareil ophtalmique selon la revendication 1, comprenant :
un premier moyen de commande d'affichage (200) servant de moyen de commande d'affichage pour afficher, sur une partie d'affichage (205), une valeur de pression intraoculaire mesurée par l'unité de mesure de pression intraoculaire et l'image fixe de l'image en coupe transversale de la zone iridocornéenne de l'œil de l'examiné photographiée par le moyen de photographie de la zone iridocornéenne.

3. L'appareil ophtalmique selon la revendication 1 ou 2, comprenant en outre :
un moyen de commande (200) pour changer de commande en fonction du mode de photographie établi par le moyen de réglage de mode,
dans lequel le moyen de commande est au moins l'un parmi :
(a) un moyen de commande pour effectuer la commande de photographie du système optique d'observation frontale (30) et du système optique d'imagerie en coupe transversale (90), le moyen de commande (a) étant configuré pour changer la commande de photographie selon le mode établi par le moyen de réglage de mode ;
(b) un moyen de commande pour effectuer une commande d'alignement d'une unité d'optométrie (4) pourvue avec le système optique d'observation frontale et le système optique d'imagerie en coupe transversale par rapport à l'œil de l'examiné, le moyen de commande (b) étant configuré pour changer la commande d'alignement selon le mode établi par le moyen de réglage de mode ;
(c) un moyen de commande pour effectuer la commande de l'unité d'affichage (205) pour afficher l'image frontale du segment antérieur et l'image en coupe transversale du segment antérieur, le moyen de commande (c) étant configuré pour changer la commande d'affichage selon le mode établi par le moyen de réglage de mode ;
(d) un moyen de commande pour effectuer un traitement d'analyse de l'image en coupe transversale du segment antérieur afin d'obtenir un paramètre de mesure relatif au segment antérieur, le moyen de commande (d) étant configuré pour commuter le traitement d'analyse selon le mode établi par le moyen de réglage de mode ; et
(e) un moyen de commande pour stocker l'image en coupe transversale du segment antérieur dans une unité de stockage (201), le moyen de commande (e) étant configuré pour stocker une partie de photographie correspondant au mode établi par le moyen de réglage de mode, en association avec l'image en coupe transversale du segment antérieur.

4. Appareil ophtalmique selon l'une quelconque des revendications 1 à 3, dans lequel le moyen de photographie de la zone iridocornéenne comprend un moyen de détection d'alignement pour détecter un état d'alignement de l'axe optique par rapport à la zone iridocornéenne de l'œil de l'examiné.

5. L'appareil ophtalmique selon la revendication 4, comprenant en outre un moyen d'alignement automatique pour ajuster une position relative de l'axe optique par rapport à la zone iridocornéenne sur la base d'un résultat de détection du moyen de détection d'alignement.

6. L'appareil ophtalmique selon l'une quelconque des revendications 1 à 5, comprenant :
un moyen d'imagerie frontale du segment antérieur pour obtenir une image fixe d'une image frontale du segment antérieur par le système optique d'observation de l'image frontale lorsque l'image en coupe transversale de la zone iridocornéenne de l'œil de l'examiné doit être photographiée ; et
un second moyen de commande d'affichage servant de moyen de commande d'affichage pour afficher, sur l'unité d'affichage, l'image en coupe transversale de la zone iridocornéenne photographiée par le moyen de photographie de la zone iridocornéenne et l'image frontale du segment antérieur photographiée par le moyen de photographie frontale du segment antérieur.

7. L'appareil ophtalmique selon l'une quelconque des revendications 1 à 6, dans lequel le moyen photographie de la zone iridocornéenne est configuré pour ajuster une position du système optique d'imagerie en coupe transversale à une position plus proche du segment antérieur de l'œil de l'examiné par rapport à une direction de distance de travail qu'une position à laquelle l'image fixe de l'image en coupe transversale de la cornée de l'œil de l'examiné par le moyen d'imagerie de la cornée.

8. L'appareil ophtalmique selon l'une quelconque des revendications 1 à 7, dans lequel le moyen de photographie de la zone iridocornéenne est configuré pour ajuster la position relative de sorte que l'axe optique soit disposé à une position dans laquelle la section de lumière est formée sur la zone iridocornéenne de l'œil de l'examiné, la position étant plus proche d'un centre cornéen de l'œil de l'examiné qu'une position de limite entre une cornée et une sclérotique de l'œil de l'examiné.

9. L'appareil ophtalmique selon l'une quelconque des revendications 1 à 8, dans lequel
le système optique d'observation frontale comprend une source de lumière visible (38),
lorsque le mode de photographie de la zone iridocornéenne est établi par le moyen de réglage de mode, la source de lumière visible est allumée pour permettre l'observation d'une image frontale visible à travers le système optique d'observation frontale.

10. L'appareil ophtalmique selon l'une quelconque des revendications 1 à 9, dans lequel
le système optique de réception de la lumière comprend un arrangement optique placé obliquement par rapport à un axe optique de projection de la lumière du système optique de projection de la lumière et basé sur une relation de Scheimpflug.

11. L'appareil ophtalmique selon l'une quelconque des revendications 1 à 10, comprenant en outre un moyen de commande d'affichage pour afficher, sur l'unité d'affichage, des informations sur l'angle de l'œil de l'examiné conjointement avec l'image fixe de l'image en coupe de la zone iridocornéenne de l'œil de l'examinateur photographiée par le moyen de photographie de la zone iridocornéenne.

12. L'appareil ophtalmique selon l'une quelconque des revendications 1 à 11, comprenant en outre un système optique de cible de fixation pour fixer une ligne visuelle de l'œil de l'examiné vers l'avant
dans lequel le moyen de photographie de la zone iridocornéenne est configuré pour obtenir l'image fixe de l'image en coupe transversale de la zone iridocornéenne de l'œil de l'examiné par le système optique d'imagerie en coupe transversale par rapport à l'œil de l'examiné fixé vers l'avant par le système optique de projection de cible de fixation.
